# EUROPÄISCHE PATENTSCHRIFT

(11) **EP 1 851 226 B1**
(45) Veröffentlichungstag und Bekanntmachung des Hinweises auf die Patenterteilung: **26.11.2014**
(21) Anmeldenummer: 06704529.4
(22) Anmeldetag: 09.02.2006
(51) Int. Cl.: C07D 489/08, A61K 31/00

(54) **VERFAHREN ZUR REINIGUNG VON NOROXYMORPHON-VERBINDUNGEN**
METHOD FOR PURIFYING NOROXYMORPHONE COMPOUNDS
PROCEDE POUR PURIFIER DES COMPOSES DE NOROXYMORPHONE

(30) Priorität: 11.02.2005 WO PCT/CH2005/000076
(43) Veröffentlichungstag der Anmeldung: 07.11.2007
(73) Patentinhaber: Cilag AG, 8205 Schaffhausen (CH)
(72) Erfinder: WEIGL, Ulrich, 78247 Hilzingen (DE); KÖTZ, Ulf, 78250 Tengen (DE); FREIFELD, Ilia, 60338 Frankfurt/Main (DE)
(74) Vertreter: Braun, André jr.
(86) Internationale Anmeldenummer: PCT/CH2006/000087
(87) Internationale Veröffentlichungsnummer: WO 2006/084412

(56) Entgegenhaltungen:
- EP-A- 0 158 476
- WO-A-91/05768
- WO-A-95/32973
- WO-A-99/02529

## Beschreibung

Die vorliegende Erfindung betrifft ein Verfahren zur Reinigung von Noroxymorphon-Verbindungen. Die vorliegende Erfindung betrifft auch ein Verfahren zur Herstellung von reinen Noroxymorphon-Verbindungen, insbesondere Naltrexon und Naloxon, insbesondere von reinem Naltrexon.

Noroxymorphon wird chemisch als 7,8-Dihydro-14-hydroxy-normorphinon oder als α,β-Dihydro-14-hydroxy-normorphinon bezeichnet und entspricht der Formel

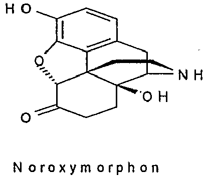

Noroxymorphon-Verbindungen und deren Herstellung sind beispielsweise in DE 272 78 05 beschrieben. Ein ausgewähltes Derivat von Noroxymorphon ist die als Naltrexon bekannte Verbindung, welche der folgenden chemischen Formel entspricht:

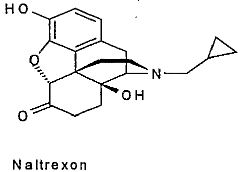

Naltrexon sowie dessen Derivate und Salze, beispielsweise Naltrexon Hydrochlorid, N-Methylnaltrexon Bromid (Methylnaltrexon) oder Naltrexone Methobromid, sind bekannte pharmazeutisch wirksame Verbindungen, die insbesondere zur Verminderung der psychischen Abhängigkeit bei Drogenmissbrauch eingesetzt werden. Naltrexon Methobromid wird beispielsweise als Antagonist des MU-Rezeptors eingesetzt, um Nebenwirkungen von Narkotika zu unterbinden. Naloxon (CAS-Nr. 465-65-5) ist am Stickstoffatom durch einen Allylrest substituiert und ist in ähnlicher Weise pharmazeutisch wirksam. Als Morphinderivate werden diese Verbindungen aus Vorstufen synthetisiert, die aus der Klasse der morphinartigen Alkaloide des Klatschmohns stammen. Da die Totalsynthese dieser komplizierten Klasse von Naturstoffen aufwendig ist, werden die Ausgangsstoffe zur Synthese von Noroxymorphon-Verbindungen aus pflanzlichen Quellen mittels Extraktion gewonnen. Bei der Extraktion von Pflanzen, im vorliegenden Fall von Mohn, wird jedoch nicht selektiv nur eine einzelne Verbindung, sondern ein Gemisch zahlreicher strukturell ähnlicher Verbindungen erhalten. Dabei sind viele dieser extrahierten Verbindungen toxisch oder ergeben im Rahmen der weiteren chemischen Umsetzung, beispielsweise bei der weiteren Synthese zu Oxymorphon, Noroxymorphon und Naltrexon, toxische Verbindungen. Als besonders problematische Verunreinigungen haben sich dabei α,β-ungesättigte Verbindungen, wie beispielsweise die Verbindung der Formeln (Ia), (Ib) und (Ic) erwiesen.

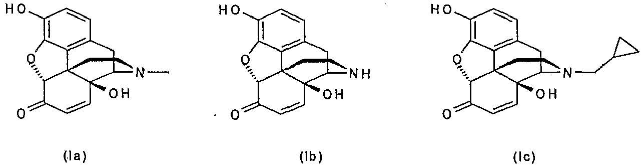

Ebenso können potentielle Vorläufer dieser Verbindungen, wie beispielsweise entsprechende α-substituierte und/oder β-substituierte Alkohole im pflanzlichen Extraktionsgemisch als Verunreinigung vorliegen, welche wiederum α,β-ungesättigte Verbindungen, wie beispielsweise die Verbindung der Formeln (Ia), (Ib) und (Ic), bilden können. Zusätzlich können weitere α,β-ungesättigte toxische Verbindungen bei der Herstellung von Naltrexon, ausgehend von den genannten pflanzlichen Extrakten, entstehen, wobei solche Verbindungen mutagen, teratogen und/ oder kanzerogen sein können. Deshalb wurden die Grenzwerte für diese Verbindungen in Naltrexon und Naltrexon Derivaten auf 100 ppm, und teilweise auf 10 ppm, herabgesetzt. Eine solche Spezifikation kann jedoch für Produkte, welche ausgehend von aus pflanzlichen Quellen extrahierten Rohstoffen nach bekannten Verfahren synthetisiert werden, in der Regel kaum erfüllt werden.

Es wurde nun gefunden, dass es gelingt, den erwähnten Grenzwert von 10 ppm für die vorgehend genannten α,β-ungesättigten Verbindungen einzuhalten bzw. zu unterschreiten, wenn man das Pflanzenextrakt, welches, neben der Noroxymorphon-Verbindung, die entsprechende α,β-ungesättigte Verbindung und weitere Verunreinigungen enthält, oder das Produkt einer nachfolgenden Stufe in der Synthese einer ausgewählten Noroxymorphon-Verbindung, (a) einer Reaktion unterwirft, durch welche die im Gemisch vorhandenen Hydroxylgruppen in Abgangsgruppen umgewandelt werden, (b) gegebenenfalls diese Abgangsgruppen wieder entfernt und anschliessend (c) das erhaltene Gemisch einer selektiven Hydrierung unterwirft.

Unter dem Ausdruck "Abgangsgruppe" ist ein Rest der Formel -OR₂ zu verstehen, wie dies im Weiteren beschrieben ist. Die "Umwandlung der im Gemisch vorhandenen Hydroxylgruppen in Abgangsgruppen" gemäss Schritt (a) bedeutet, dass die im Gemisch vorhandenen Hydroxylgruppen in Reste der Formel -OR₂, umgewandelt werden, worin R₂ den eingeführten Rest bedeutet. Die "Entfernung der Abgangsgruppen" gemäss Schritt (b) bedeutet, dass man den eingeführten Rest R₂ aus den Resten der Formel -OR₂ entfernt.

Dabei wird die Aufarbeitung von Schritt (a) und von Schritt (b) inklusive einer möglichen Isolierung der Reaktionsprodukte vorzugsweise in nicht-wässerigem Medium, vorzugsweise auch in nicht-alkoholischem Medium, durchgeführt. Bevorzugt ist die Entfernung der Abgangsgruppen vor der Hydrierung. Die Hydrierung, d.i. Schritt (c), kann in Gegenwart von nicht-protischen und unter milden Bedingungen auch in Gegenwart von protischen Lösungsmitteln, wie Wasser und Alkoholen, durchgeführt werden. Nach der Hydrierung können zusätzlich allenfalls noch vorhandene Abgangsgruppen mittels Hydrolyse entfernt werden.

Durch diese Umwandlung der im Gemisch vorhanden Hydroxylgruppen in Abgangsgruppen [Schritt (a)] und gegebenenfalls anschliessender Entfernung dieser Abgangsgruppen [Schritt (b)], werden bei der Hydrierung [Schritt (c)] sämtliche kritischen Verunreinigungen, welche üblicherweise in der Grössenordnung von etwa 1000 ppm in den Ausgangsprodukten vorhanden sind, soweit entfernt, dass diese mittels HPLC analytisch nicht mehr nachweisbar sind.

Besonders überraschend ist, dass durch die erfindungsgemässe Vorbehandlung des Rohproduktes, d.h. des pflanzlichen Extraktes, die Hydrierung derart selektiv wirkt, dass sämtliche kritische Nebenprodukte praktisch gänzlich entfernt werden, wobei in den Noroxymorphon-Verbindungen aus den Abgangsgruppen wieder die gewünschten Hydroxylgruppen gebildet werden, ohne dass die vorhandene Ketogruppe hydriert bzw. entfernt oder in eine Hydroxylgruppe umgewandelt wird. Solch hohe Reinheiten lassen sich durch einfache Hydrierung des Rohgemisches nicht erreichen. Vermutlich werden potentielle Vorläufer der Noroxymorphon-Verbindung, wie beispielsweise entsprechende α-substituierte und/oder β-substituierte Alkohole, welche im pflanzlichen Extraktionsgemisch als Verunreinigung vorliegen, durch die erfindungsgemässe Umsetzungen gemäss Schritt (a) oder den Schritten (a) und (b) derart verändert, dass diese oder Folgeprodukte (z.B. Eliminationsprodukte) aus diesen Umsetzungen durch die Hydrierung in Schritt (c) in Methylengruppen umgewandelt werden. Die vorliegende Erfindung ist aber nicht an diese Erklärung gebunden.

Erfindungsgemäss kann auch beispielsweise bei der Herstellung von Noroxymorphon oder bei dessen weiteren Verarbeitung zu Naltrexon oder Naloxon und deren Salze, entweder das Ausgangsgemisch oder eine beliebige Zwischenstufe oder auch das Endprodukt, d.i. Naltrexon oder Naloxon, vorzugsweise das Ausgangsgemisch oder eine Zwischenstufe, der erfindungsgemässen Behandlung gemäss Schritt (a) und Schritt (b) unterworfen und anschliessend hydriert werden.

Das Ausgangsgemisch besteht in der Regel aus Oxymorphon, 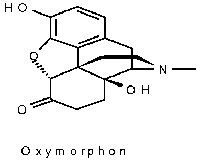 welches aus den aus pflanzlichen Materialien extrahierten Naturstoffen Thebain oder Oripavin hergestellt wurde.

Die vorliegende Erfindung betrifft ein Verfahren zur Herstellung von Noroxymorphon aus einer rohen Zusammensetzung, welche ein Pflanzenextrakt darstellt oder aus pflanzlichen Extrakten gewonnen wurde, welche im wesentlichen aus Noroxymorphonverbindungen der Formel (II) bestehen und welche als Verunreinigungen mindestens eine in α,β-Stellung ungesättigte Noroxymorphonverbindungen der Formeln (Ia) und (Ib) sowie weitere verunreinigende Noroxymorphonverbindungen enthält: 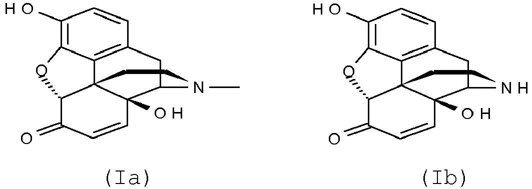 wobei die Noroxymorphonverbindungen der Formel (II) der folgenden Formel entsprechen: 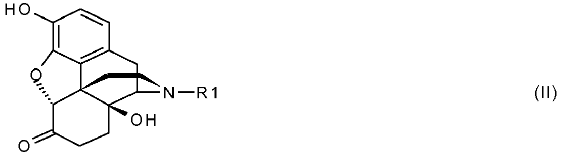 worin
R₁, Wasserstoff, oder Methyl, wie solche im Pflanzenextrakt vorhanden sind, oder einen eingeführten (C₁-C₄)-Alkyloxycarbonyl-, Phenyloxycarbonyl-, oder Cyclohexyloxycarbonylrest bedeutet, dadurch gekennzeichnet, dass man (a) die Zusammensetzung in einer Reaktion umsetzt, durch welche die im Gemisch vorhandenen Hydroxylgruppen in Reste der Formel -OR₂ umgewandelt werden, worin R₂ den eingeführten Rest bedeutet, und der Rest der Formel -OR₂ eine Estergruppierung darstellt, worin R₂ vorzugsweise Formylcarbonyl, Methylcarbonyl, Trichlormethylcarbonyl, Trifluormethylcarbonyl, Phenylcarbonyl, gegebenenfalls substituiertes Phenylcarbonyl bedeutet, oder eine Estergruppierung einer Sulfonsäure darstellt, worin R₂ Methylsulfonyl, Benzylsulfonyl oder p-Toluoylsulfonyl bedeutet, oder der Rest der Formel -OR₂ eine Kohlensäureestergruppierung darstellt, worin R₂ (C₁-C₈)-Alkyloxycarbonyl oder Phenyloxycarbonyl bedeutet, (b) den eingeführten Rest R₂ aus den Resten der Formel -OR₂ wieder entfernt, (c) das erhaltene Gemisch einer selektiven Hydrierung unterwirft, so dass in α,β-Stellung der verunreinigenden Noroxymorphonverbindungen eine gesättigte Bindung entsteht und gegebenenfalls vorhandene Reste der Formel -OR₂ in eine Hydroxylgruppe umgewandelt werden, wobei man diese selektive Hydrierung in Gegenwart von nicht-protischen oder in protischen Lösungsmitteln durchführt und (d) die reine Noroxymorphonverbindung isoliert.

Die vorliegende Erfindung betrifft auch die nach dem erfindungsgemässen Verfahren gereinigten Oxymorphonverbindungen der Formel (II) oder ein Gemisch solcher Verbindungen sowie pharmazeutische Formulierungen enthaltend eine solche Verbindung.

R₁ bedeutet vorzugsweise Wasserstoff, (C₁-C₈) -Alkyl, (C₂-C₄) -Alkenyl oder eine Abgangsgruppe; vorzugsweise (C₁-C₆) -Alkyl, Allyl oder Wasserstoff, vorzugsweise (C₁-C₆)-Alkyl oder Wasserstoff.

R₁ als Abgangsgruppe bedeutet vorzugsweise (C₁-C₄)-Alkyloxycarbonyl [(C₁-C₈)-Alkyl-O-C(O)-] oder Phenyloxycarbonyl [Phenyl-O-C(O)-], vorzugsweise Ethyloxycarbonyl, Isobutyloxycarbonyl, oder tert.-Butyloxycarbonyl (Boc), Cyclohexyloxycarbonyl, vorzugsweise Ethyloxycarbonyl oder tert.-Butyloxycarbonyl (Boc). Für die Einführung des Restes geht man in an sich bekannter Weise vor, indem man die Verbindung der allgemeinen Formel (II) (worin R₁ Wasserstoff oder einen ersetzbaren Rest bedeutet), beispielsweise mit Boc-Anhydrid (Boc-O-Boc) {[(CH₃)₃C-O-C(O)]₂-O} oder mit Boc-Carbamat [(CH₃)₃C-O-C(O)-N(C₁₋₄-Alkyl)₂], umsetzt. Solche Reste und deren Einführung an Stickstoffstoffatome sind an sich bekannt.

Stellt die Verbindung der Formel (II) ein Endprodukt dar, so bedeutet darin R₁ vorzugsweise Methylen-cyclopropyl (-CH₂-C₃H₅) oder Allyl (-CH₂-CH=CH₂). Vorzugsweise wird eine Verbindung bzw. ein Verbindungsgemisch hydriert, worin R₁ weder Methylen-cyclopropyl noch Allyl bedeutet und man aus dem hydrierten Produkt das bevorzugte Endprodukt herstellt.

Die Definition "im wesentlichen aus Noroxymorphonverbindungen der Formel (II) bestehend und welche als Verunreinigungen in α,β-Stellung ungesättigte Noroxymorphonverbindungen sowie weitere verunreinigende Noroxymorphonverbindungen enthalten", bedeutet, dass die pflanzlichen Extrakte als Feststoff gesamthaft etwa mindestens 70 Gew.-%, vorzugsweise mindestens 80 Gew.-% und vorzugsweise mindestens etwa 90 Gew.-% an Noroxymorphonverbindungen enthalten, wobei das Verhältnis von Noroxymorphonverbindungen der Formel (II) zu den verunreinigenden Noroxymorphonverbindungen etwa im Bereich von 99.800 Gew.% bis 99.999 Gew.% Noroxymorphonverbindungen der Formel (II) zu etwa 0.200 Gew.% bis 0.001 Gew.% an verunreinigenden Verbindungen liegt, und sämtliche im Extrakt anwesenden Feststoffe zusammen 100 Gew.-% ergeben.

In der Abgangsgruppe der Formel -OR₂, bildet -OR₂ vorzugsweise eine Estergruppierung, wie beispielsweise den Formylesterrest [R₂ = HC(O)-], Acetylesterrest [R₂ = CH₃C(O)-, Methylcarbonyl], Trichloracetylesterrest [R₂ = CCl₃C(O)-], Trifluoracetylesterrest [R₂ = CF₃C(O)-, Trifluormethylcarbonyl], Benzoylesterrest [R₂ = C₆H₅C(O)-], gegebenenfalls substituierte Benzylestergruppen, oder Ester von Sulfonsäuren, worin R₂ vorzugsweise Methylsulfonyl, Benzylsulfonyl oder p-Toluoylsulfonyl, bedeutet. Alternativ kann -OR₂ auch eine Kohlensäureestergruppierung bilden, worin R₂ (C₁-C₈)-Alkyloxycarbonyl oder Phenyloxycarbonyl bedeutet; vorzugsweise Ethyloxycarbonyl, Isobutyloxycarbonyl, oder tert.-Butyloxycarbonyl (Boc), Cyclohexyloxycarbonyl, vorzugsweise Ethyloxycarbonyl oder tert.-Butyloxycarbonyl (Boc).

Für die Bildung einer Estergruppierung, z.B. bei der Einführung von Acetyl oder tert.-Butyloxycarbonyl (Boc), geht man in an sich bekannter Weise vor, dass man die Verbindung der allgemeinen Formel (II) mit Acetanhydrid oder Acetylchlorid bzw. Boc-Anhydrid (Boc-O-Boc) {[(CH₃)₃C-O-C(O)]₂-O}, umsetzt. Dabei stehen hier Acetyl und Boc stellvertretend für die anderen gleich reagierenden Verbindungen, das heisst Verbindungen, worin der Methyl bzw. der tert.-Butylrest ersetzt ist durch einen andern gleich reagierenden Rest. Die Abgangsgruppen werden in der Regel im Verlauf der Umsetzung entfernt, z.B. in Schritt (b) oder bei der Hydrierung, doch können diese nach der Hydrierung zusätzlich in an sich bekannter Weise entfernt werden, sollte dies fallweise nötig sein.

Bevorzugt ist die Einführung einer Abgangsgruppe, bzw. Derivatisierung, mittels Umsetzung mit Säurechloriden oder Säureanhydriden, wie beispielsweise Acetanhydrid, Acetylchlorid, Trifluoressigsäureanhydrid, Methansulfonylchlorid, Methansulfonylanhydrid, Toluolsulfonylchlorid, und verwandte an sich bekannte Verbindungen.

Die Umwandlung von R₁ in eine Abgangsgruppe, falls es sich bei R₁ um eine Alkylgruppe handelt, ist aus analogen Umsetzungen aus der Literatur bekannt und braucht hier nicht weiter beschrieben zu werden.

Vorzugsweise wird die weitere Umsetzung des in Stufe (a) erhaltenen Reaktionsgemisches in wasserfreiem Medium, vorzugsweise auch in alkoholfreiem Medium, vorgenommen, da sich durch die Anwesenheit von Wasser Verunreinigungen bilden können, insbesondere Alkohole in α- oder β-Stellung durch die Addition von Wasser und gegebenenfalls Alkohol an α,β-Stellung ungesättigten Verbindungen. Dies gilt für die Aufarbeitung von Stufe (a) und Entfernung der Abgangsgruppen gemäss Stufe (b), inklusive einer möglichen Isolierung der Reaktionsprodukte. Die Hydrierung gemäss Stufe (c) kann unter milden Bedingungen in wässrigen und/- oder alkoholischen Lösungsmitteln durchgeführt werden. Für solche Behandlungen in wasserfreien und vorzugsweise alkoholfreien Medien sind insbesondere nicht-protische Lösungsmittel, wie z.B. tert.-Butylether, geeignet.

Die Abgangsgruppe gemäss Stufe (a) wird durch Behandeln des Reaktionsgemisches, gegebenenfalls unter Erwärmen, mit einem Acylierungsmittel, wie vorgehend beschrieben, eingeführt. Durch anschliessende Zugabe von organischen Lösungsmitteln, z.B. MTBE (Methylter.-butylether), wird das Produkt ausgefällt.

Für die Entfernung der Abgangsgruppen gemäss Stufe (b) geht man vorzugsweise so vor, dass man das Reaktionsprodukt aus Stufe (a) in nicht wässrigen Lösungsmitteln, gegebenenfalls während mehreren Stunden, erhitzt, vorzugsweise in nicht-protischen Lösungsmitteln, wie THF, Dioxan, Ethylacetat, MTBE, DMF, DMSO und dergleichen, gegebenenfalls unter Zusatz einer Base, wie Kalium-tert.-butylat oder Lithiumhydroxid in nicht-protischen Lösungsmitteln, wie beispielsweise THF, Dioxan, oder Ethylacetat. Bevorzugt wird das Produkt anschliessend durch Zugabe eines nicht-protischen Lösungsmittels ausgefällt.

Hydrierungsbedingungen sind an sich bekannt und z.B. in EP 0 158 476, WO99/02529, WO 95/32973 oder O 91/05768 erwähnt. Bevorzugt sind erfindungsgemäss Hydrierungsbedingungen, worin für die Hydrierung in Stufe (c) elementarer Wasserstoff und/oder Bedingungen bzw. Verbindungen, welche elementaren Wasserstoff *in situ* generieren, verwendet werden. In diesem Sinne sind erfindungsgemäss Hydrierungsbedingungen bevorzugt, worin für die Hydrierung in Stufe (c) elementarer Wasserstoff, Cyclohexen und/oder Cyclohexadien (welche unter Wasserstoffabgabe zu Benzol reagieren) und/oder Ammoniumformiat (welches unter Wasserstoffabspaltung zu Kohlendioxid und Ammoniak zerfällt) als Wasserstoffquellen oder in einem Lösungsmittel aus der Klasse der polaren organischen Lösungsmittel, gegebenenfalls unter Zusatz von Wasser zur Lösungsvermittlung, wie beispielsweise Hydrierungskatalysatoren, verwendet werden. Solche Hydrierungskatalysatoren sind hierin weiter unten beschrieben. Transferhydrierungen können in der Regel bei Normaldruck durchgeführt werden und sind an sich bekannt.

Insbesondere bevorzugt ist die katalytische Hydrierung unter Verwendung von Edelmetallkatalysatoren in heterogener oder homogener Form. Solche Edelmetallkatalysatoren sind vorzugsweise ausgewählt aus Verbindungen der Gruppe der Übergangsmetalle des Periodensystems der Elemente, insbesondere ausgewählt aus Metallen der VIII. Gruppe des Periodensystems, deren Verbindungen und Komplexe, insbesondere von Ruthenium (Ru) und Osmium (Os), Cobalt (Co), Rhodium (Rh), und Iridium (Ir), Nickel (Ni), Palladium (Pd) und Platin (Pt). Bevorzugt sind Rhodium, Palladium und Platin, insbesondere Palladium. Diese Metalle werden als Hydrierungskatalysatoren in an sich bekannter Weise verwendet. So kann an in heterogener Form hydrieren, wobei die Katalysatoren auf einem Trägermaterial aufgetragen sind, vorzugsweise auf Aktivkohle oder Aluminiumoxid oder auf anderen an sich bekannten Trägermaterial, vorzugsweise auf Aktivkohle.

Bevorzugt können auch Verbindungen dieser Metalle als homogene Katalysatoren verwendet werden, vorzugsweise Palladiumverbindungen. Beispiele für solche Palladiumverbindungen sind an sich bekannte Pd(O)-Verbindungen wie Tetrakis(triphenylphosphin)-palladium sowie die entsprechenden Komplexe mit den Liganden Tri-(2-tolyl)phosphin, Tri-(2-furyl)phosphine, Tri(tert.-butyl)phosphin, bzw. den zweizähnigen Liganden dppm [1,1-Bis (diphenylphosphinomethan)], dppe [1,2-Bis-(diphenylphosphino)-ethan] und verwandte Verbindungen, und Tris(dibenzylidenaceton)diPalladium-ChloroformKomplex, sowie Pd(II)-Verbindungen wie PdCl₂, Pd (dppe) Cl₂, Pd (OAc)₂, Pd (dppe) (OAc)₂, π-Allyl-Pd-Komplexe, vorzugsweise π-Allyl-Pd-chlorid Dimer. Bevorzugt sind Pd(0)-Verbindungen. Diese Verbindungen, bzw. Salze und Komplexe, sind an sich bekannt und in der Literatur beschrieben worden.

Die Katalysatoren werden in katalytischen Mengen, vorzugsweise in Mengen von 0.0005 - 0.01 Gew.-% Edelmetall, vorzugsweise etwa 0.001 - 0.005 Gew.-% Edelmetall, berechnet auf das Gewicht des Rohdukts eingesetzt. Die angegebene obere Grenze ist aber nicht kritisch. So können auch höhere Mengen an Katalysator, z.B. äquimolare Mengen bezogen auf das Rohprodukt, eingesetzt werden. In der Regel ist dies jedoch nicht nötig.

Die Hydrierung wird vorzugsweise mit Wasserstoffgas durch geführt, vorzugsweise in einem inerten Lösungsmittel, wie beispielsweise in organischen Säuren, wie vorzugsweise Eisessig, Ameisensäure, Propionsäure oder einem Gemisch dieser Verbindungen; in Alkoholen, wie vorzugsweise Methanol, Ethanol, Isopropylalkohol, n-Butanol oder ein Gemisch dieser Verbindungen; in Nitrilen wie vorzugsweise Acetonitril und/oder Propionitril; in Ketonen wie vorzugsweise Aceton und/oder 2-Butanon; in Estern wie Essigssäureethylester, in polaren aprotischen Lösungsmitteln wie vorzugsweise Dimethylformamid (DMF) oder Dimethylsulfonamid (DMSO), gegebenenfalls unter Wasserzusatz. Bevorzugt sind protische Lösungsmittel, insbesondere Methanol, Ethanol, Isopropylalkohol, n-Butanol, oder aprotische polare Lösungsmittel, vorzugsweise Aceton, DMF, Acetonitril, gegebenenfalls im Gemisch mit 1-99 Gew.-% Wasser und vorzugsweise in Gegenwart einer organischen Säure, wie beispielsweise Essigsäure, Trifluoroessigsäure, Propionsäure, Ameisensäure, vorzugsweise Essigsäure, vorzugsweise in einer Konzentration von 0.1 Gew.-% bis 99 Gew.-%. Die Hydrierung wird vorzugsweise bei einer Temperatur im Bereich von 0°C bis 150°C, bevorzugt im Bereich von 20°C bis 100°C, vorzugsweise im Bereich von Normaldruck bis 100 bar, vorzugsweise im Bereich von Normaldruck bis 10 bar, durchgeführt.

Anstelle von Wasserstoff können auch Verbindungen verwendet, welche in der Reaktion *in situ* Wasserstoff abgeben, wie beispielsweise Transferhydrierung mit Ammoniumformiat, Cyclohexen und/oder Cyclohexadien. Dabei wird der Wasserstoff in einer vorgelagerten Reaktion unter Katalyse von Reagenz abgespalten.

Die vorliegende Erfindung betrifft auch ein Verfahren zur Herstellung von reinem Noroxymorphon aus pflanzlichen Extrakten, welche im wesentlichen aus Noroxymorphon bestehen und welche verunreinigende Noroxymorphonverbindungen enthalten, dadurch gekennzeichnet, dass man Oxymorphon der vorgehend genannten Formel (II), worin R₁ Methyl bedeutet, als Pflanzenextrakt vorlegt, und (a) das Pflanzenextrakt in einer Reaktion umsetzt, durch welche die im Gemisch vorhandenen Hydroxylgruppen in Abgangsgruppen der Formel -OR₂ umgewandelt werden, worin R₂ den eingeführten Rest einer Abgangsgruppe bedeutet, wie solche vorgehend für R₁ beschrieben sind, vorzugsweise Acyl, vorzugsweise Acetyl;
(a1) die N-Methylgruppe [entsprechend der Bedeutung von R₁ der Verbindung der Formel (II)] entfernt und durch eine Abgangsgruppe R₃ ersetzt, worin R₃ eine Abgangsgruppe bedeutet, wie solche vorgehend für R₁ beschrieben sind, vorzugsweise Alkyloxycarbonyl, vorzugsweise Ethyloxycarbonyl oder Boc, vorzugsweise Ethyloxycarbonyl;
(a2) gegebenenfalls aus dem Reaktionsprodukt erhalten aus den Stufen (a) und (a1) die Abgangsgruppen R₂ und R₃ entfernt;
(b) mindestens eines der in den Stufen (a), (a1) und/oder (a2) erhaltenen Produkte, vorzugsweise eines der in den Stufen (a1) oder (a2), vorzugsweise in Stufe (a2), erhaltenen Produkte, einer selektiven Hydrierungsreaktion unterwirft, wie diese vorgehend beschrieben ist, und
(c) gegebenenfalls die reine Noroxymorphon-Verbindung isoliert.

Das in Stufe (a2) erhaltene Produkt kann auch weiter verarbeitet werden, vorzugsweise zu Naltrexon oder Naloxon oder einem Salz dieser Verbindungen oder einem quaternären Derivat dieser Verbindungen, vorzugsweise zum Hydrochlorid, Hydrobromid, Methochlorid oder Methobromid, vorzugsweise zu den entsprechenden Salzen oder quaternären Derivaten von Naltrexon. Durch die selektive Hydrierung werden auch die Abgangsgruppen entfernt, doch können diese gegebenenfalls separat in Stufe (a2) und/oder zur Vervollständigung, insofern nötig, im Anschluss an die Hydrierung, durchgeführt werden.

In Stufe (a) wird Oxymorphon vorzugsweise mittels Acetanhydrid in Methyl-tert.-butylether (MTBE) verestert und wasserfrei aufgearbeitet und isoliert, wobei Diacetyloxymorphon (R₂ = Acetyl) erhalten wird.

In Stufe (a1) wird vorzugsweise mittels Chlorameisensäureethylester in einem aprotischen Lösungsmittel umgesetzt, vorzugsweise Acetonitril, unter basischen Bedingungen, wie mit K₂CO₃, demethyliert, wobei eine Oxymorphonverbindung isoliert wird, worin R₃ Ethoxycarbonyl bedeutet, bzw. die erhaltene Verbindung das entsprechende Diacetyloxymorphon-ethoxycarbamat darstellt.

In Stufe (a2) werden die Abgangsgruppen R₂ und R₃ aus dem Reaktionsprodukt, erhalten aus den Stufen (a) und (a1), entfernt. Hierzu erhitzt man das Reaktionsprodukt aus Stufe (a) oder (a1) in nicht-wässrigen Lösungsmitteln, vorzugsweise in nicht-protischen Lösungsmitteln wie THF, Dioxan, Ethylacetat, MTBE, DMF, DMSO und dergleichen, gegebenenfalls über mehrere Stunden, gegebenenfalls unter Zusatz einer Base wie Kalium-tert.-butylat oder Lithiumhydroxid, in nicht-protischen Lösungsmitteln wie beispielsweise THF, Dioxan, Ethylacetat. Bevorzugt wird durch Zugabe eines nicht-protischen Lösungsmittels das Produkt anschliessend ausgefällt.

In Stufe (b) wird das isolierte Produkt, z.B. Diacetyloxymorphon-ethoxycarbamat, vorzugsweise in Eisessig gelöst und durch Einleiten von Wasserstoffgas unter den vorgehend angegebenen Bedingungen, katalysiert durch Palladium auf Aktivkohle, einer Hydrierung unterworfen. Anschliessend werden die verbliebenen Abgangsgruppen R₄ und R₅ durch Zugabe von 40%-iger Schwefelsäure zum Reaktionsgemisch abgespalten, wobei sich Noroxymorphon-Sulfat bildet, welches gegebenenfalls isoliert werden kann. Durch Basenzugabe, beispielsweise durch Zugabe von Ammoniaklösung in Ethanol/Wasser, kann das Reaktionsgemisch neutralisiert und aufgearbeitet werden, wobei das freie Noroxymorphon isoliert werden kann. Das freie Noroxymorphon ist unlöslich in einem Wasser/Ethanolgemisch bei einem schwach alkalischen Säurewert (pH-Wert), bevorzugt pH 8-10, und fällt bei der pH-Wert Einstellung als kristalliner Feststoff aus, wodurch es abfiltriert werden kann. Im isolierten Noroxymorphon sind mittels HPLC keine α,β-ungesättigten Verbindungen mehr nachweisbar. Das derart erhaltene Noroxymorphon kann somit weiter verarbeitet werden, vorzugsweise zu hochreinem Naltrexon oder Naloxon (CAS-Nr. 465-65-5) oder zu Salzen oder quaternären Derivaten. Als Salze sind die Hydrochloride und Hydrobromide bevorzugt. Als quaternäre Derivate sind die Verbindungen Naltrexon Methobromid (wird auch als Methylnaltrexon bezeichnet) oder Naloxon Methobromid [wird auch als Methylnaloxon bezeichnet (CAS-Nr. 73232-50-5)] bevorzugt. Bevorzugt sind Naltrexon Hydrochloride oder Hydrobromide und Naltrexon Methobromid.

Das erfindungsgemäss hergestellte Noroxymorphon kann z.B. zu hochreinem Naltrexon oder hochreinem Naloxon, oder zu einem hochreinem Salz oder quaternären Derivat dieser Verbindungen, verarbeitet werden.

In diesem Sinne betrifft die vorliegende Erfindung ein Verfahren zur Herstellung von hochreinem Salzen und quaternären Derivaten von Naltrexon und Naloxon, bei welchen die kritischen olefinischen Verunreinigungen unterhalb der Nachweisgrenze liegen, vorzugsweise Salze von Naltrexon, indem man das Ausgangsprodukt Noroxymorphon mit dem entsprechenden Alkylierungsmittel, d.i. mit Cyclopropylmethylbromid (für Naltrexon) oder mit Allylbromid (für Naloxon) umsetzt und das Produkt Naltrexon oder Naloxon entweder mit einer Säure, vorzugsweise mit verdünnter Salzsäure oder Bromwasserstoff zum entsprechenden Salz umsetzt, im beschriebenen Fall zum Hydrochlorid bzw. Hydrobromid; oder mit einem weiteren Alkylierungsmittel behandelt, vorzugsweise mit Methylbromid, wobei man Naltrexon Methobromid bzw. Naloxon Methobromid erhält; dadurch gekennzeichnet, dass man mindestens das Ausgangsprodukt oder ein als Zwischenstufe erhaltenes Produkt der Stufen (a) oder (b) oder das Endprodukt, vorzugsweise ein als Zwischenstufe erhaltenes Produkt der Stufen (a) oder (b), vorzugsweise der Stufe (b) einer Hydrierungsreaktion unterwirft, wie diese vorgehend beschrieben ist. Die folgenden Beispiele erläutern die Erfindung.

### Beispiel 1 [Herstellung von Diacetyloxymorphon (DAOM), Einführung der Abgangsgruppe mit direkter Eliminierung der Abgangsgruppe]

20 g Oxymorphon werden in einer Mischung aus 10 g tert.-Butylmethylether und 21 g Essigsäureanhydrid (3.24 eq.) bei Raumtemperatur suspendiert. Die Reaktionslösung wird 5 Stunden unter Rückfluss erhitzt. Danach wird abgekühlt und 70 g tert.-Butylmethylether zugegeben. Die Suspension wird nochmals auf Rückflusstemperatur erhitzt, dann auf 0-4°C abgekühlt und bis zur vollständigen Fällung nachgerührt. Das Produkt wird abgesaugt, mit tert.-Butylmethylether nachgewaschen und bei 90°C im Vakuum bis zur Gewichtskonstanz getrocknet. Ausbeute: 23 g (91 %, bezogen auf das eingesetzte Oxymorphon); HPLC-Reinheit: 98%, Produkt enthält in Spuren (ca. 1000 ppm) α,β-ungesättigte Verbindung; 3,8,14-Triacetyloxymorphon ist nicht nachweisbar.

### Beispiel 2 [Herstellung von Diacetyloxymorphon (DAOM), welches in Spuren 3,8,14-Triacetyloxymorphon enthält; Einführung einer Abgangsgruppe]

20 g Oxymorphon werden in einer Mischung aus 10 g tert.-Butylmethylether und 21 g Essigsäureanhydrid (3.24 eq.) bei Raumtemperatur suspendiert. Die Reaktionslösung wird 48 Stunden bei max 30-40°C erhitzt. Danach wird abgekühlt und 70 g tert.-Butylmethylether zugegeben. Es wird auf 0-4°C abgekühlt und bis zur vollständigen Fällung nachgerührt. Das Produkt wird abgesaugt, mit tert.-Butylmethylether nachgewaschen und bei 30°C im Vakuum bis zur Gewichtskonstanz getrocknet. Ausbeute: 26,8 g (91 %, bezogen auf das eingesetzte Oxymorphon); HPLC-Reinheit: 98%, Produkt enthält in Spuren 3,8,14-Triacetyloxymorphon.

### Beispiel 3 (Herstellung von Diacetyloxymorphon-Carbamat)

30g Diacetyloxymorphon werden zusammen mit 66g Chlorameisensäureethylester (8 eq.) und einer heterogenen Base (Kaliumcarbonat 1 eq.) in einem organischen Lösungsmittel (74 g Acetonitril) suspendiert und mehrere Stunden (24-28 Stunden) bei erhöhter Temperatur (65-68°C) erhitzt. Nach Beendigung der Reaktion werden Acetonitril und Chlorameisensäureethylester unter Vakuum abdestilliert. Zum Rückstand werden 73 g Acetonitril dazugegeben. Bei Raumtemperatur wird dann die heterogene Base (KHCO₃/K₂CO₃) abfiltriert. Das Acetonitril wird unter Vakuum abdestilliert und zur vollständigen Fällung 60 g tert.-Butylmethylether zugegeben. Nach Erhitzen auf Rückflusstemperatur wird auf 0-5°C abgekühlt und nachgerührt, danach der ausgefallene Feststoff abgesaugt und zuerst mit tert.-Butylmethylether, danach mit Wasser gewaschen. Das farblose Produkt wird bei 80°C bis zur Gewichtskonstanz im Vakuum getrocknet. Gemäss HPLC enthält das Produkt >1000 ppm α,β-ungesättigte Verbindungen.

Ausbeute: 29 g (86% bezogen auf das eingesetzte Diacetyloxymorphon). HPLC-Reinheit: >95%.

### Beispiel 4 [Umsetzung von 3,14-Diacetyloxymorphon (DAOM), das in Spuren 3,8,14-Triacetyloxymorphon enthält, zu 3,8,14-Triacetyloxymorphon-freiem 3,14-Diacetyloxymorphon (DAOM); Eliminierung der Abgangsgruppe]

20 g Diacetyloxymorphon mit Spuren 3,8,14-Triacetyloxymorphon werden in einer Mischung aus 20 g tert.-Butylmethylether und 3-5 g Essigsäure bei Raumtemperatur suspendiert. Die Reaktionslösung wird 10-15 Stunden bei 70°C erhitzt. Danach wird abgekühlt und 70 g tert.-Butylmethylether zugegeben. Es wird auf 0-4°C abgekühlt und bis zur vollständigen Fällung nachgerührt. Das Produkt wird abgesaugt, mit tert.-Butylmethylether nachgewaschen und bei 30°C im Vakuum bis zur Gewichtskonstanz getrocknet. Ausbeute: 15.7 g (91 %, bezogen auf das eingesetzte Diacetyloxymorphon); HPLC-Reinheit: 98%, Produkt enthält ca. 1000 ppm α,β-ungesättigte Verbindung, 3,8,14-Triacetyloxymorphon ist nicht nachweisbar.

### Bespiel 5 (Hydrierung mit Abgangsgruppe)

20 g Diacetyloxymorphon mit Spuren 3,8,14-Triacetyloxymorphon, hergestellt gemäss Beispiel 2, werden in 60 g Eisessig bei Raumtemperatur gelöst. Es werden 0.6g wasserfeuchtes Palladium auf Aktivkohle (10% Pd bezogen auf die Trockensubstanz, Wassergehalt ca. 50%) dazugegeben. Dann wird bei 50-60°C Innentemperatur und 2.7 bar Wasserstoffgas eingeleitet. Nach der Hydrierung wird der Katalysator abfiltriert und die Lösung wird unter Vakuum auf die Hälfte aufkonzentriert. Anschliessend wird MTBE zugegeben und auf 0-4°C gekühlt. Das Produkt wird abgesaugt, mit MTBE nachgewaschen und im Vakuum bei 70°C getrocknet. Reinheit: 98%; weder α,β-ungesättigte Verbindung noch 3,8,14-Triacetyloxymorphon sind nachweisbar, Ausbeute 85% (Diacetyloxymorphon bezogen auf eingesetztes Diacetyloxymorphon)

### Beispiel 6 (Hydrierung mit eliminierter Abgangsgruppe; Herstellung von Noroxymorphon)

30 g Diacetyloxymorphon-Carbamat, hergestellt nach Beispiel 3, werden in 60 g Eisessig bei Raumtemperatur gelöst. Es werden 0.6g wasserfeuchtes Palladium auf Aktivkohle (10% Pd bezogen auf die Trockensubstanz, Wassergehalt ca. 50%) dazugegeben. Dann wird bei 50-60°C Innentemperatur und 2.7 bar Wasserstoffgas eingeleitet. Nach der Hydrierung wird der Katalysator abfiltriert und die Lösung wird unter Vakuum auf die Hälfte aufkonzentriert. Zur aufkonzentrierten Eisessiglösung wird das dreifache Volumen 40ige% Schwefelsäure zugegeben. Unter Rückfluss wird das Carbamat zum freien Amin verkocht. Dabei fällt das Produkt als Sulfatsalz aus. Das entstandene Salz wird abfiltriert und mit wenig gekühltem Ethanol nachgewaschen. Der erhaltene Feststoff wird in Wasser/Ethanol gelöst und die Lösung mit wässriger Ammoniaklösung auf einen pH-Wert von 9 (neun) gebracht. Bei diesem pH-Wert fällt das freie Noroxymorphon aus und wird abfiltriert. Mittels HPLC-Analyse sind keine α,β-ungesättigte Nebenprodukte mehr nachweisbar. Ausbeute: 70-75% (bezogen auf das eingesetzte Diacetyloxymorphon-Carbamat);

HPLC-Reinheit: >98%, weder α,β-ungesättigte Verbindung noch 3,8,14-Triacetyloxymorphon nachweisbar.

## Patentansprüche

1. Verfahren zur Herstellung von Noroxymorphon aus einer rohen Zusammensetzung, welche ein Pflanzenextrakt darstellt oder aus pflanzlichen Extrakten gewonnen wurde, welche im wesentlichen aus Noroxymorphonverbindungen der Formel (II) bestehen und welche als Verunreinigungen mindestens eine in α,β-Stellung ungesättigte Noroxymorphonverbindungen der Formeln (Ia) und (Ib) sowie weitere verunreinigende Noroxymorphonverbindungen enthält: 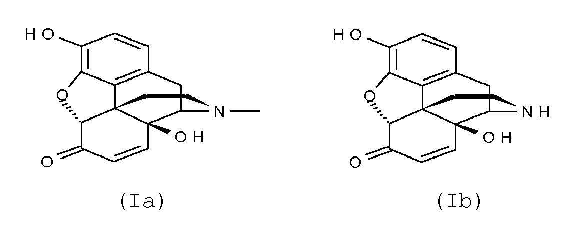 wobei die Noroxymorphonverbindungen der Formel (II) der folgenden Formel entsprechen: 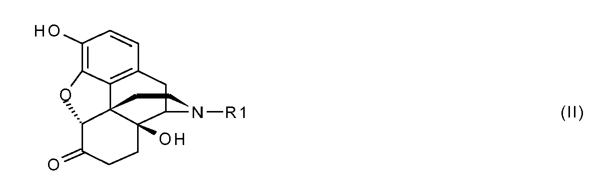 worin
R₁, Wasserstoff, oder Methyl, wie solche im Pflanzenextrakt vorhanden sind, oder einen eingeführten (C₁-C₄)-Alkyloxycarbonyl-, Phenyloxycarbonyl-, oder Cyclohexyloxycarbonylrest bedeutet, **dadurch gekennzeichnet, dass** man (a) die Zusammensetzung in einer Reaktion umsetzt, durch welche die im Gemisch vorhandenen Hydroxylgruppen in Reste der Formel -OR₂ umgewandelt werden, worin R₂ den eingeführten Rest bedeutet, und der Rest der Formel -OR₂ eine Estergruppierung darstellt, worin R₂ vorzugsweise Formylcarbonyl, Methylcarbonyl, Trichlormethylcarbonyl, Trifluormethylcarbonyl, Phenylcarbonyl, gegebenenfalls substituiertes Phenylcarbonyl bedeutet, oder eine Estergruppierung einer Sulfonsäure darstellt, worin R₂ Methylsulfonyl, Benzylsulfonyl oder p-Toluoylsulfonyl bedeutet, oder der Rest der Formel -OR₂ eine Kohlensäureestergruppierung darstellt, worin R₂ (C₁-C₈)-Alkyloxycarbonyl oder Phenyloxycarbonyl bedeutet, (b) den eingeführten Rest R₂ aus den Resten der Formel -OR₂ wieder entfernt, (c) das erhaltene Gemisch einer selektiven Hydrierung unterwirft, so dass in α,β-Stellung der verunreinigenden Noroxymorphonverbindungen eine gesättigte Bindung entsteht und gegebenenfalls vorhandene Reste der Formel -OR₂ in eine Hydroxylgruppe umgewandelt werden, wobei man diese selektive Hydrierung in Gegenwart von nicht-protischen oder in protischen Lösungsmitteln durchführt und (d) die reine Noroxymorphonverbindung isoliert.

2. Verfahren nach Anspruch 1, **dadurch gekennzeichnet, dass** man die eingeführten Reste R₂ aus den Resten der Formel -OR₂ vor der selektiven Hydrierung [Schritt (c)] entfernt.

3. Verfahren nach Anspruch 1, **dadurch gekennzeichnet, dass** man die Aufarbeitung von Schritt (a), von Schritt (b) sowie von Schritt (c) in nicht-wässerigem Medium und vorzugsweise auch in nicht-alkoholischem Medium durchführt.

4. Verfahren nach einem der Ansprüche 1-3, **dadurch gekennzeichnet, dass** das in der selektiven Hydrierung [Schritt (c)] verwendete nicht-protische Lösungsmittel eine Esterverbindung, vorzugsweise Essigsäureethylester und das protische Lösungsmittel, vorzugsweise Wasser und/oder einen Alkohol, darstellt.

5. Verfahren nach einem der Ansprüche 1-4, **dadurch gekennzeichnet, dass** man nach der Hydrierung [Schritt (c)] noch vorhandene Reste R₂ aus den Resten der Formel -OR₂ mittels Hydrolyse entfernt.

6. Verfahren nach einem der Ansprüche 1-5, **dadurch gekennzeichnet, dass** R₁ Wasserstoff oder Methyl bedeutet.

7. Verfahren nach einem der Ansprüche 1-5, **dadurch gekennzeichnet, dass** R₁ Ethyloxycarbonyl, Isobutyloxycarbonyl oder tert.-Butyloxycarbonyl (Boc) bedeutet.

8. Verfahren nach einem der Ansprüche 1-5, **dadurch gekennzeichnet, dass** -OR₂ eine Kohlensäureestergruppierung, worin R₂ Ethyloxycarbonyl, Isobutyloxycarbonyl, oder tert.-Butyloxycarbonyl (Boc), Cyclohexyloxycarbonyl, vorzugsweise Ethyloxycarbonyl oder tert.-Butyloxycarbonyl (Boc) bedeutet.

9. Verfahren nach einem der Ansprüche 1-9, **dadurch gekennzeichnet, dass** die Zusammensetzung als Feststoff gesamthaft mindestens 70 Gew.-%, vorzugsweise mindestens 80 Gew.-% und vorzugsweise mindestens 90 Gew.-% an Noroxymorphonverbindungen enthält, wobei das Verhältnis der Noroxymorphonverbindungen der Formel (II) zu den verunreinigenden Noroxymorphonverbindungen im Bereich von 99.800 Gew.% bis 99.999 Gew.% an Noroxymorphonverbindungen der Formel (II) zu 0.200 Gew.% bis 0.001 Gew.% an verunreinigenden Verbindungen liegt, und sämtliche im Extrakt anwesenden Feststoffe zusammen 100 Gew.-% ergeben.

10. Verfahren nach einem der Ansprüche 1-9, **dadurch gekennzeichnet, dass** man das in Stufe (c) oder Stufe (d) erhaltene Noroxymorphon zu Naltrexon oder Naloxon oder einem Salz dieser Verbindungen oder einem quaternären Derivat dieser Verbindungen, vorzugsweise zum Hydrochlorid, Hydrobromid, Methochlorid oder Methobromid, vorzugsweise zu Naltrexon Hydrochlorid, Naltrexon Hydrobromid, Naltrexon Methochlorid oder Naltrexon Methobromid, weiter verarbeitet.

11. Verfahren nach einem der Ansprüche 1-10, **dadurch gekennzeichnet, dass** die Konzentration der in α,β-Stellung ungesättigten verunreinigenden Noroxymorphonverbindungen in der gemäss Schritt(d) erhaltenen reinen Noroxymorphonverbindung weniger als 10 ppm beträgt.

## Claims

1. Method for preparing noroxymorphone from a crude composition which represents a plant extract or which has been obtained from plant extracts composed essentially of noroxymorphone compounds of formula (II), and which as an impurity contains at least one noroxymorphone compound of formulas (Ia) and (Ib) which is unsaturated in the α,β-position, as well as other noroxymorphone compounds as impurities: 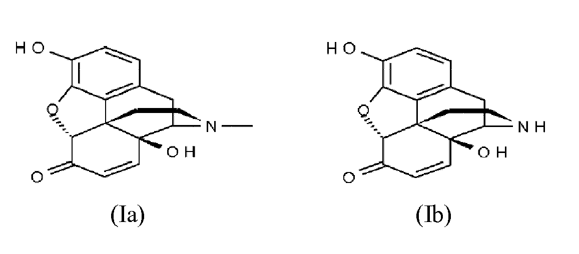 wherein the noroxymorphone compounds of formula (II) correspond to the following formula: 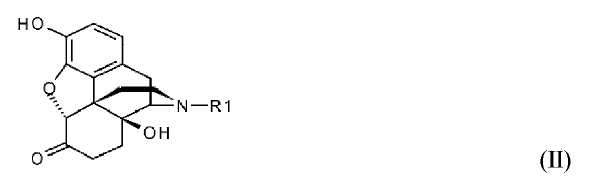 where
R₁ stands for hydrogen or methyl such as present in the plant extract, or an introduced (C₁-C₄) alkyloxy carbonyl, phenyloxy carbonyl, or cyclohexyloxy carbonyl radical, **characterized in that** (a) the composition is reacted in a reaction by means of which the hydroxyl groups present in the mixture are converted into radicals of formula -OR₂, where R₂ stands for the introduced radical, and the radical of formula -OR₂ represents an ester group, where R₂ preferably stands for formyl carbonyl, methyl carbonyl, trichloromethyl carbonyl, trifluoromethyl carbonyl, phenyl carbonyl, or optionally substituted phenyl carbonyl, or represents an ester group of a sulfonic acid, where R₂ stands for methyl sulfonyl, benzyl sulfonyl, or *p*-toluene sulfonyl, or the radical of formula -OR₂ represents a carbonic acid ester group, where R₂ stands for (C₁-C₈) alkyloxy carbonyl or phenyloxy carbonyl, (b) the introduced radical R₂ is removed from the radicals of formula -OR₂, (c) the obtained mixture is subjected to selective hydrogenation, so that a saturated bond results at the α,β-position of the noroxymorphone compound impurities, and radicals of formula -OR₂ which may be present are converted into a hydroxyl group, this selective hydrogenation being carried out in the presence of aprotic solvents or in protic solvents, and (d) the pure noroxymorphone compound is isolated.

2. Method according to Claim 1, **characterized in that** the introduced radicals R₂ are removed from the radicals of formula -OR₂ prior to the selective hydrogenation (step (c)).

3. Method according to Claim 1, **characterized in that** the workup of step (a), of step (b), and of step (c) is carried out in nonaqueous medium, and preferably also in nonalcoholic medium.

4. Method according to one of Claims 1 through 3, **characterized in that** the aprotic solvent used in the selective hydrogenation (step (c)) represents an ester compound, preferably ethyl acetate, and the protic solvent preferably represents water and/or an alcohol.

5. Method according to one of Claims 1 through 4, **characterized in that** radicals R₂ which are still present after the hydrogenation (step (c)) are removed from the radicals of formula -OR₂ by hydrolysis.

6. Method according to one of Claims 1 through 5, **characterized in that** R₁ stands for hydrogen or methyl.

7. Method according to one of Claims 1 through 5, **characterized in that** R₁ stands for ethyloxy carbonyl, isobutyloxy carbonyl, or *tert*-butyloxy carbonyl (Boc).

8. Method according to one of Claims 1 through 5, **characterized in that** -OR₂ stands for a carbonic acid ester group, where R₂ stands for ethyloxy carbonyl, isobutyloxy carbonyl, or *tert*-butyloxy carbonyl (Boc), cyclohexyloxy carbonyl, preferably ethyloxy carbonyl or *tert*-butyloxy carbonyl (Boc).

9. Method according to one of Claims 1 through 9, **characterized in that** the composition as a solid contains overall at least 70% by weight, preferably at least 80% by weight, and preferably at least 90% by weight, of noroxymorphone compounds, wherein the ratio of the noroxymorphone compounds of formula (II) to the noroxymorphone compound impurities is in the range of 99.800% by weight to 99.999% by weight of noroxymorphone compounds of formula (II) to 0.200% by weight to 0.001 % by weight of impurity compounds, and the total of all solids present in the extract is 100% by weight.

10. Method according to one of Claims 1 through 9, **characterized in that** the noroxymorphone obtained in step (c) or step (d) is further processed to produce naltrexone or naloxone or a salt of these compounds or a quaternary derivative of these compounds, preferably the hydrochloride, hydrobromide, methochloride, or methobromide, preferably naltrexone hydrochloride, naltrexone hydrobromide, naltrexone methochloride, or naltrexone methobromide.

11. Method according to one of Claims 1 through 10, **characterized in that** the concentration of the noroxymorphone compound impurities, which are unsaturated at the α,β-position, in the pure noroxymorphone compound obtained according to step (d) is less than 10 ppm.

## Revendications

1. Procédé de fabrication de noroxymorphone à partir d'une composition brute qui présente un extrait végétal ou qui a été obtenue à partir d'extraits végétaux qui se composent essentiellement de composés de la noroxymorphone de formule (II), et qui contient comme impuretés au moins un des composés de la noroxymorphone insaturés en position α-β de formules (Ia) et (Ib), ainsi que d'autres composés de la noroxymorphone comme impuretés : 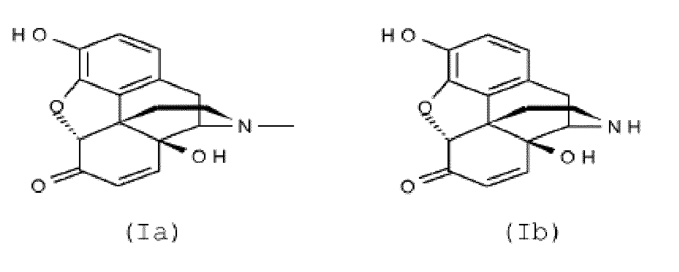 où les composés de la noroxymorphone de formule (II) correspondent à la formule suivante : 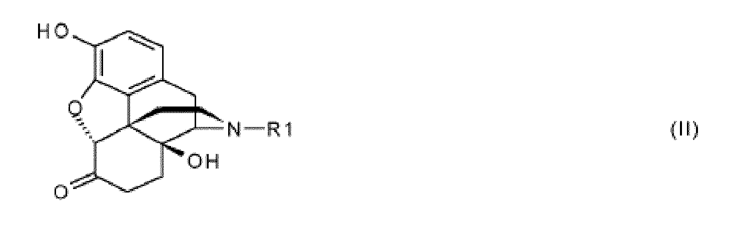 où :
R1 signifie hydrogène ou méthyle, comme présent dans l'extrait végétal, ou un résidu introduit (C₁-C₄)-alkyloxycarbonyle, phényloxycarbonyle ou cyclohexyloxycarbonyle, caractérisé (a) en ce qu'on convertit la composition par une réaction où les groupes hydroxyles présents dans le mélange sont convertis en résidus de formule -OR₂, où R2 représente le résidu introduit, et où le résidu de formule -OR₂ représente un groupe d'estérification, où R2 représente préférentiellement formylcarbonyle, méthylcarbonyle, trichlorométhylcarbonyle, trifluorométhylcarbonyle, phénylcarbonyle, éventuellement phénylcarbonyle substitué, ou un groupe d'estérification d'un acide sulfonique, où R2 représente méthylsulfonyle, benzylsulfonyle ou p-toluoylsulfonyle, ou le résidu de formule -OR₂ représente un groupe de carbonatation où R2 représente (C₁-C₈)-alkyloxycarbonyle ou phényloxycarbonyle, (b) en ce qu'on élimine le résidu introduit R2 des résidus de formule -OR₂, (c) en ce qu'on soumet le mélange obtenu à une hydrogénation sélective de façon qu'en position α-β des composés impuretés de la noroxymorphone contiennent une liaison saturée et à ce que les résidus éventuellement présents de formule -OR₂ soient convertis en un groupe hydroxyle, où l'on effectue l'hydrogénation sélective en présence de solvants aprotiques ou protiques, et (d) en ce qu'on isole le composé noroxymorphone pur.

2. Procédé selon la revendication 1, **caractérisé en ce qu'**on élimine le résidu introduit R₂ du résidu de formule -OR₂ avant l'hydrogénation sélective [étape (c)].

3. Procédé selon la revendication 1, **caractérisé en ce qu'**on traite l'étape (a), l'étape (b) ainsi que l'étape (c) dans un milieu non-aqueux, et préférentiellement aussi dans un milieu non-alcoolique.

4. Procédé selon l'une des revendications 1-3, **caractérisé en ce que** le solvant aprotique utilisé dans l'hydrogénation sélective [étape (c)] présente une liaison ester, préférentiellement l'acétate d'éthyle, et **en ce que** le solvant protique est préférentiellement de l'eau et/ou un alcool.

5. Procédé selon l'une des revendications 1-4, **caractérisé en ce que** les résidus R₂ encore présents après l'hydrogénation [étape (c)] sont éliminés des résidus de formule -OR₂ par hydrolyse.

6. Procédé selon l'une des revendications 1-5, **caractérisé en ce que** R₁ représente un hydrogène ou un méthyle.

7. Procédé selon l'une des revendications 1-5, **caractérisé en ce que** R1 représente un éthyloxycarbonyle, un isobutyloxycarbonyle ou un tertiobutyloxycarbonyle (Boc).

8. Procédé selon l'une des revendications 1-5, **caractérisé en ce que** -OR₂ représente un groupe carbonate, où R₂ représente un éthyloxycarbonyle, isobutyloxycarbonyle, tertiobutyloxycarbonyle (Boc), cyclohexyloxycarbonyle, préférentiellement éthyloxycarbonyle ou tertiobutyloxycarbonyle (Boc).

9. Procédé selon l'une des revendications 1-9, **caractérisé en ce que** la composition contient comme solide en tout au moins 70 % en poids, préférentiellement au moins 80 % en poids et préférentiellement au moins 90 % en poids de composés de la noroxymorphone, et le rapport des composés de la noroxymorphone de formule (II) par rapport aux impuretés de la noroxymorphone se situe entre 99,800 % en poids et 99,999 % en poids en composés de la noroxymorphone de formule (II) et de 0,200 % en poids à 0,001 % en poids d'impuretés, et toutes les substances présentes dans l'extrait représentent ensemble 100 % en poids.

10. Procédé selon l'une des revendications 1-9, **caractérisé en ce qu'**on retraite la noroxymorphone obtenue à l'étape (c) ou à l'étape (d) en naltrexone ou en naloxone ou en un sel de ces composés ou en un dérivé quaternaire de ces composés, préférentiellement en chlorhydrate, bromhydrate, chlorure de méthyl ou bromure de méthyl, préférentiellement en chlorhydrate de naltrexone, bromhydrate de naltrexone, chlorure de méthylnaltrexone ou bromure de méthylnaltrexone.

11. Procédé selon l'une des revendications 1-10, **caractérisé en ce que** la concentration des impuretés insaturées en position α-β de la noroxymorphone dans le composé noroxymorphone pur obtenu à l'étape (d) est inférieure à 10 ppm.
